# EUROPEAN PATENT SPECIFICATION

(11) **EP 0 737 461 B1**
(45) Date of publication and mention of the grant of the patent: **08.12.1999**
(21) Application number: 96302566.3
(22) Date of filing: 11.04.1996
(51) Int. Cl.: A61F 13/04

(54) **Scrim reinforced orthopaedic casting tape**
Mit einem Netz verstärktes Bord für Steifverbände
Ruban pour plâtre orthopédique, renforcé par un filet

(30) Priority: 12.04.1995 US 421346
(43) Date of publication of application: 16.10.1996
(73) Proprietor: JOHNSON & JOHNSON PROFESSIONAL Inc., Raynham, Massachusetts 02767-0350 (US)
(72) Inventor: Fitzpatrick, Brian A., Randolph, MA 02368 (US); Weaver, Gregory A., Duxbury, MA 02331 (US); Vanschalkwijk, Kurt, Andover, MA 01810 (US)
(74) Representative: Fisher, Adrian John

(56) References cited:
- FR-A- 1 323 119
- US-A- 4 668 563
- US-A- 5 334 446

## Description

### FIELD OF THE INVENTION

The present invention relates to an orthopedic casting tape composed of a highly extensible non-woven fabric impregnated or coated with water hardenable prepolymer resin. The non-woven fabric comprises an open mesh thermoplastic scrim, preferably elastic, and staple fibers affixed to at least one side of the scrim.

### BACKGROUND OF THE INVENTION

Orthopedic casting tapes or bandage materials have been used for many years to immobilize broken limbs. The earliest casting material was plaster of Paris which was made by depositing plaster of Paris on a mesh fabric. The mesh fabric containing the plaster of Paris was sold in the form of a roll which would be dipped into water and then applied to the patient. The plaster of Paris would harden after contacting the water and would eventually develop sufficient strength to immobilize and support the limb of the patient. Although these bandages were adequate to immobilize the limb, they had a very low strength to weight ratio and were quite heavy. They also would disintegrate if they were excessively wetted with water. Later casting tapes were made with water curable polyurethane prepolymers coated on knitted substrates. The knitted substrates gave some extensibility and conformability when the casting tapes were applied. Bandages of this construction are described in U. S. Patents 4,411,262; 4,376,438; 4,502,479 and 4,433,680. The substrates for these bandages were knitted from polyester or fiberglass yarns, although other yarns are also suggested. In U.S. Patent 4,668,563 there is disclosed a knitted substrate which contains elastomeric threads in the longitudinal direction to give the casting tape extensibility and recovery which provides better conformability to the casting tape when the casting tape is applied to the limb of a patient.

Although the casting tapes described in the patents mentioned above are more than adequate to provide the intended function, the knitting process is quite slow and, therefore, the knitted substrates are somewhat costly and increase the cost of the casting tapes. Non-woven fabrics have been used as substrates in plaster of Paris casting bandages and are disclosed in U.S. Patent 3,972,323. U. S. Patents 4,841,958 and 4,856,502 disclose polyurethane casting tapes made with a non-woven fabric containing a number of apertures in the fabric. The non-woven fabric substrates of these patents have an extensibility of 10 to about 45 percent, but have no recovery, that is, after the force necessary to stretch the fabric is relaxed, the fabric does not return to its unstretched length. These substrates are less expensive than knitted substrate and are extensible, but do not recover after they have been stretched, and do not have the strength and conformability of knitted fabrics. A composite non-woven fabric made from an elastic scrim and a fibrous web hydroentangled with the scrim is disclosed in U.S. Patent 5,334,446.

### SUMMARY OF THE PRESENT INVENTION

The present invention provides a low cost, non-woven cast substrate which has improved strength when compared to previously proposed non-woven cast substrates. The non-woven substrates of the present invention include a thermoplastic scrim secured, interlaced or affixed to a web of bonded or unbonded fibers. The scrim reinforced non-woven fabric is coated with a water reactive polymer, such as polyurethane, to form the casting tape which is packaged in a moisture-impervious package until use.

The present invention may also provide a non-woven substrate with significant extensibility and recovery in the length direction of the tape and yet is a relatively low cost substrate. This is accomplished by using a scrim made from an elastomeric polymer. The elastomeric polymers used in the scrim provide extensibility and recovery to the substrate which results in improved conformability in the casting tape.

Casts made employing the substrates of the present invention are equivalent in strength to casts made with knitted polyester substrates.

### BRIEF DESCRIPTION OF THE DRAWINGS:

Fig 1 is a perspective view of the elastomeric scrim used in the present invention.
Fig 2 is a perspective view of the scrim with fibers applied to both sides of the scrim.
Fig. 3 is a graph of strength versus weight of various casting tape products including the tape of the present invention.
Fig. 4 is an illustration of the process employed to needle punch the web of fibers to the scrim.

### DETAILED DESCRIPTION OF THE INVENTION

As mentioned above, the substrate of the present invention combines a thermoplastic scrim with or without a fiber web to form a non-woven substrate for orthopedic casting tape. The scrim is formed from a thermoplastic polymer by a variety of different techniques. The net or scrim can be extruded, see for example, U.S. Patent 4,410,587; the scrim can be formed from an embossed sheet see for example U. S. Patent 4,434,199 or they can be formed by other techniques. U.S. Patents 3,773,606; 3,881,381; 4,013,752; 4,173,612; 4,285,998; 4,305,990; 4,329,309; 4,381,326; 4,434,199; 4,410,587; 4,416,718; and 4,755,247 disclosed extensible net like materials and processes for making such products. The product of the type disclosed in U.S. Patent 5,334,446 comprising an elastic scrim and hydroentangled fibers may also be employed as the substrate.

The particular technique to make the net or scrim is not critical if the scrim has the desired properties. Thermoplastic scrims can come in a variety of geometric shapes or patterns and hole openings. For the purpose of this invention, the geometric shapes that are preferred are the square and the rectangle patterns of openings. These shapes may be cut parallel to the machine direction to maximize utilization of the fabric. It should be noted that other shapes are also available i.e., diamond and hexagonal, and that the stretch of the fabric is attained by the extensibility of the elastomer i.e., filament as well as the elongation of the geometric shape. The scrim should have a weight of between 0,016 and 0,08 kg per m² (0.5 and 2.5 ounces per square yard), a thickness of from 0,127 to 12,7 mm (0.005 to 0.50 inches) and should have between 58 and 5800 opening per cm² (9 and 900 openings per square inch).

If a non-elastic polymer is used, some extensibility can be obtained by the selection of the geometric shape, e.g. a diamond pattern in the scrim will allow some extensibility.

The elastomeric thermoplastic materials that are used in the manufacture of the scrim products can be individual thermoplastic elastomers, referred to as TPEs or blends of resins which have different properties. There is usually what is termed a hard resin segment and a soft resin segment in such blends. Examples of such TPEs are styrene block copolymers made with polystyrene and isoprene and/or polybutadiene such as materials known as KRATON™; materials made from polyester and polyether known as HYTREL™; materials made from polyurethanes and polyether such as the materials known as PELLATHANE™; materials made from polyamide and polyether such as the materials known as PEBAX™. Examples of elastomeric blends are materials made from polypropylene and ethylene propylene rubbers, the materials known as SANTOPRENE™ and, copolymers of an ethylene and a comonomer such as butene, hexene, octene, decene or 4-methylpentene and are available as EXACT™ plastomers from Exxon Chemical which can be used by themselves or blended with a polyolefin resin such as polyethylene or polypropylene. These plastomers are disclosed in U.S. Patents 5,382,630 and 5,382,631. All of these materials have the potential to be used in the present invention. Typical of the EXACT™ plastomers useful in the present invention are grades EXACT™ 4049, EXACT™ 4041, EXACT™ 4033, EXACT™ 4011, and EXACT™ 4015. Non-elastomeric resins that can be used include polyolefins such as polyethylene and polypropylene, polyesters and polyamides.

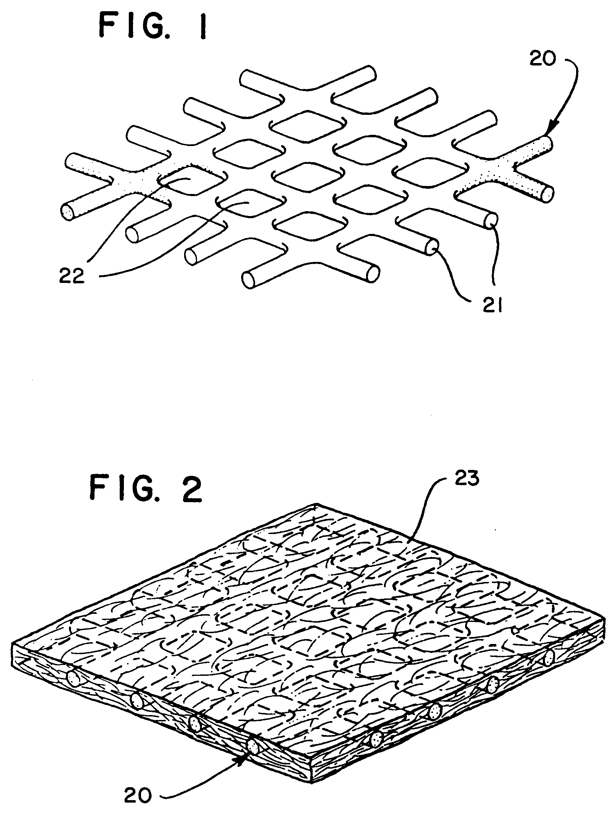

A suitable scrim configuration for both elastomeric and non-elastomeric resins is illustrated in Fig. 1. The scrim 20 is composed of thermoplastic filaments 21 which are arranged in a square pattern with openings 22 between the intersecting filaments. Fig. 2 illustrates a scrim with in fibers 23 secured to the scrim.

In the manufacture of the composite substrate of the present invention, a scrim is formed by any of the processes mentioned above and using the above mentioned resins and a layer of fibers is secured to one or both surfaces of the scrim. The fibers can be any fiber which is compatible with the polyurethane resin such as polyester, polypropylene or polyamide fibers. Other fibers that can be used are carbon, rayon, acetate, acrylic, modacrylic, glass, aramid and natural fibers. Blends of such fibers may also be used. These fibers generally have a denier of between 1 and 100 dpf, preferably between 1 and 15, and a length of between 0,635 and 10,16 cm (0.25 and 4 inches). The preferred fibers are staple fibers having a length of 2,54 and 1,42 cm (1 and 9/16 inches). The fibers can be formed in a batt by any of the dry laid or wet laid web forming processes commonly employed in forming such webs or batts. The batt or web of fibers is secured to the elastic scrim by any technique that will secure the fibers to the elastomeric filaments in the scrim with sufficient strength so that the fibers will not separate from the elastic scrim when the substrate is stretched.

The preferred method of securing the fiber web to the scrim is to place a web of fibers in contact with at least one surface of scrim and feed the web and scrim through a needleloom and needling or needlepunching the fibers in the web through the openings in the scrim to secure the web to the scrim. Standard needlelooms are employed. The needles are generally from 32 to 40 gauge and from 6,35 to 10,16 cm (2 1/2 to 4 inches) long. The needle density is from 90 to 164 needles per square inch and the loom is operated at about 600 strokes per minute. Following the needlepunching, the combined or composite web may be post treated by calendering, coating or other post treating techniques.

The needlepunching technique also provides some filaments that are oriented in the Z direction of the fabric and which project beyond the plane of the major surface of the fabric. The orientation of some fibers in the Z direction of the fabric adds strength to wrapped layers of the coated casting tape made from the substrate. The projections of fibers from the surface of the fabric provides improved resistance to delamination of adjacent layers of the casting tape as suggested in U.S. Patent 5,273,802.

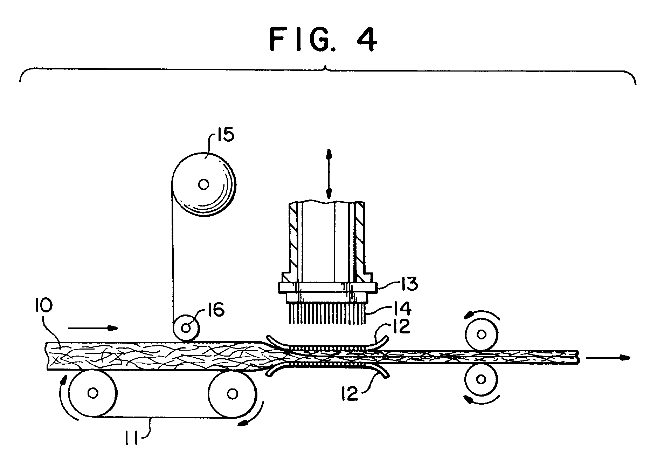

The process of securing the scrim to the fiber web is shown in Fig. 4. A batt or web of non-woven fibers 10 is fed an a moving belt 11 to a needleloom. The batt is compressed to some extent as it is fed through the screens 12. There is a needle board 13 which carries a plurality of barbed needles 14. The board containing the needles is reciprocated in an up and dawn motion to move the needles 14 through the web. There is a roll of scrim 15 which is of substantially the same width as the web 10, fed onto the web around roller 16. The needles 14 act to move, by a pushing and pulling action, the fibers in the web 10 through the scrim 15 to entangle the fibers around the filaments of the scrim to form the unitary composite web shown in Fig. 2. The total weight of the substrate fabric is generally between 0,05 and 0,23 kg/m² (1.5 and 7 oz./square yard) and preferably between 0,066 - 0,166 kg/m² (2-5 oz/square yard). The substrate with an elastomeric scrim should have an extensibility of between 5% and 100% depending on the load, and a recovery of at least 65%.

It is also possible to use adhesively bonded non-woven fabrics in place of the unbonded batt of fibers. If a colored or patterned casting tape is desired, the fibers may be dyed in any color or the substrates may be printed with suitable dyes that are compatible with the water hardenable resin.

The water hardenable polyurethane prepolymer are the reaction products of aromatic isocyanates and polyols. The aromatic isocyanates useful in the prepolymer system of the present invention may be any of the aromatic polyisocyanates known in polyurethane chemistry which are described, for example, in the "Polyurethanes, Chemistry and Technology," Part I, Interscience Publishers (1962).

The aromatic polyisocyanates preferred include toluene diisocyanate (TDI), such as the 90/20 or the 65/35 isomer mixture of the 2,4 and 2,6 isomeric forms; diphenylmethane diisocyanate (MDI), such as the 4,4', and 2,4' and the 2,2' isomeric forms or isomeric mixtures thereof; modified MDI containing additional functional groups such as carbodiimide groups, urethane groups and allophanate groups and polymethylene polyphenylisocyanates (Polymeric MDI) which are derived from phosgenation of the condensation products of aniline and formaldehyde. The most preferred polyisocyanate is the carbodiimide containing MDI which is readily available commercially, e.g. Isonate 2143L.

The polyols useful in the prepolymer systems of the present are polyether polyols which are made by the polymerization of propylene oxide or combinations of ethylene oxide and propylene oxide in which the propylene oxide is present in a high enough quantity to result in a hydrophobic polyol. Hydrophilic polyols may also be used. These are combinations of ethylene oxide and propylene oxide in which the ethylene oxide is present in sufficient quantity to result in a hydrophilic product.

Generally, the molecular weight range of the polyols useful in the present invention are between 200 and 6,000 and preferably between 600 and 2,000. The polyols have a functionality, i.e., OH group of at least 2 and preferably 2 or 3.

The preferred polyurethane prepolymer used in the present invention in made from diphenylmethanediisocyanate reacted with a polyol containing two or three functional groups. The preferred polyol are those disclosed in U.S. Patent 4,433,680.

The ratio of the polyisocyanate to the polyol in the prepolymer is best expressed by the equivalent ratio. Equivalent weight is determined by dividing the molecular weight of each particular component by its functionality or number of functional groups in the compound. The equivalent ratio is the ratio of the equivalency of the isocyanate to the polyol in the composition. The equivalent ratio in the present system should be between 2:1 to approximately 15:1 equivalents of the polyisocyanate to the polyol and preferably from 2:1 TO 10:1. The components are combined so that there is an excess of from 5% to 30% NCO groups in the prepolymer.

The prepolymer formulations of the present invention will also contain from 01.% to about 10%, by weight based on the total weight of the mixture, of a catalyst. The catalyst may be an amine such as dimethylethanolamine (DMEA) or a mixture of DMEA and bis(2-dimethylaminoethyl) ether or a dimorpholinodialkylether. The preferred catalyst is the dimorpholinodiethylether catalyst disclosed in U.S. Patent 4,433,680.

In addition to the polyisooyanate, the polyol and the catalyst, the prepolymer may also include a small amount, i.e. 0.01% to 1% by weight of a stabilizer such as benzoylchloride.

In addition to water hardenable polyurethanes other hardenable resin systems can also be used with this substrate. Examples include light activated systems as disclosed in U.S. Patent 3,421,501; 3,881,473 and 4,512,340. Chemically activated systems such as U.S. Patent 3,215,137; 3,630,194; 3,618,599 and 4,134,397 and other hardenable resin systems may also be used.

The water hardenable polyurethane prepolymer is coated or impregnated onto the substrate in sufficient amounts to insure the formation of a rigid weight supporting cast. Generally, the add-on of the prepolymer is from about 35% to 80% by weight of the total weight of the cast. The preferred add-on is between 65% and 75%. The polyurethane prepolymer or other water hardenable material can be coated or impregnated onto the substrate using any suitable procedure.

### EXAMPLE I

An elastomeric scrim having a weight of 0,05 kg/m² (1.5 ounces per square yard) was combined with a 0,1 kg/m² (three ounce per square yard) air laid batt of 10,16 cm (four inch) long, 6 dpf polyester fiber. The polyester batt was secured to the scrim by needling the batt with 36 gage needles 7,62 to 8,89 cm (3 to 3 1/2 inches) long. The elongation of the scrim reinforced batts was determined in both the machine direction (MD) cross direction (CD) of the bath by applying 203,2 gr/cm (80 grams per inch) width of fabric or a weight of 1737,3 gr/cm (684 grams per inch) width of fabric. The results are shown in Table I.

**Table I**

| Weight | % Elongation | |
|---|---|---|
| | MD | CD |
| 80 grams | 5.3 | 8.3 |
| 684 grams | 65 | 46 |

### EXAMPLE II

The scrim reinforced batt of Example I was compressed by calendaring under minimal pressure. The extensibility was determined in the same manner as in Example I. The results are shown in Table II.

**TABLE II**

| Weight | % Elongation | |
|---|---|---|
| | MD | CD |
| 80 grams | 5.7 | 7.7 |
| 684 grams | 38 | 38 |

### EXAMPLE III

An elastomeric scrim having a weight of 0,066 kg/m² (2 ounces per square yard) was combined with a 0,1 kg/m² (three ounce per square yard) air laid batt of four inch long 6 dpf polyester fibers. The polyester batt was secured to the scrim by needling the batt as in Example I. The machine direction and cross direction elongation were determined in the same manner as in Example I. The results are given in Table III.

**TABLE III**

| Weight | % Elongation | |
|---|---|---|
| | MD | CD |
| 80 grams | 5.3 | 6.6 |
| 684 grams | 66 | 43 |

### EXAMPLE IV

The scrim reinforced batt of Example III was compressed by the application of minimal pressure at room temperature. The extensibility was determined in the same manner as in Example III. The results are shown in Table IV.

**TABLE IV**

| Weight | % Elongation | |
|---|---|---|
| | MD | CD |
| 80 grams | 2.3 | 5.9 |
| 684 grams | 18 | 33 |

### EXAMPLE V

Scrim reinforced batts of Example (II and IV) were coated with a polyurethane prepolymer to an add on of 71.38%. A length of the casting tape to be tested is dipped in water at 23,9 °C (75°F) and the bandage is squeezed 4 or 5 times under the surface of the water. A test cylinder is then formed by wrapping layers of the tape around a 6,98 cm (2 3/4 inch) wooden or metal dowel. The test cylinders are removed from the dowel and aged for different time periods. The samples are compressed and the load necessary to deflect the test cylinder a distance of one centimeter is determined as is the reported crush strength. In these Examples, the crush strength is reported on samples aged 24 hours. The crush strength in pounds and the weight of the test cylinder is reported.

The resulting casting tapes had an extensibility of 24% in the length direction when tested with a weight of 684 grams per inch width. The crush strengths of test cylinders made with varying number of layers of the tape is shown in Table V.

**TABLE V**

| | Example II Substrate | | Example IV Substrate | |
|---|---|---|---|---|
| Layers | Strength of Cylinder kg (pounds) | Weight of Cylinder (grams) | Strength of Cylinder kg (pounds) | Weight of Cylinder (grams) |
| 8 | 65,6 (144.7) | 72.65 | 47,9 (105.7) | 63.91 |
| 5 | 27,7 (61.24) | 50.15 | 28,1 (62.01) | 47.40 |
| 4 | 16,6 (36.58) | 40.36 | 16,2 (35.80) | 35.82 |
| 3 | 10,8 (23.99) | 33.05 | 7,2 (16.02) | 24.47 |

### EXAMPLE VI

A 0,1 kg/m² (3.0 ounce per square yard) polyester batt was needled to a 0,05 kg/m² (1.5 ounce per square yard) elastomeric scrim. The polyester was a staple fiber with a length of 4 inch and a dpf of 6. The scrim reinforced batt was coated with a water hardenable polyurethane prepolymer. The add-on was targeted for between 65 - 75%. A test cylinder was formed by wetting the coated reinforced scrim and wrapping three to eight layers of the tape around a 6,98 cm (2 3/4 inch) diameter dowel. The samples were aged for 24 hours prior to the crush evaluation. The crush strength was determined as in Example V.

**Table VI**

| Layers | Strength of Cylinder kg (pounds) |
|---|---|
| 8 | 109,4 (241.3) |
| 5 | 34,2 (75.4) |
| 4 | 23,0 (50.8) |
| 3 | 11,7 (25.9) |

### Example VII

A 0,08 kg/m² (2.5 ounce per square yard) polyester batt was needled to a 0,036 kg/m² (1.1 ounce per square yard) elastomeric scrim. The polyester was a staple fiber with a length of 10,16 cm (4 inches) and a dpf of 6. The scrim reinforced batt was coated with a water hardenable polyurethane prepolymer. The add-on was targeted for between 65 - 75%. A test cylinder was formed by wetting the coated reinforced scrim and wrapping three to eight layers of the tape around a 6,98 cm (2 3/4 inch) diameter dowel. The samples were aged for 24 hours prior to the crush evaluation. The crush strength was determined as in Example V.

**Table VII**

| Layers | Strength of Cylinder kg (pounds) |
|---|---|
| 7 | 48,2 (106.40) |
| 5 | 26,9 (59.50) |
| 4 | 18,3 (40.48) |

### Example VIII

A 0,033 kg/m² (1.0 ounce per square yard) polyester batt was needled to a 0,036 kg/m² (1.1 ounce per square yard) elastomeric scrim. The polyester was a random blend of 2 to 6 dpf staple fiber. The scrim reinforced batt was coated with a water hardenable polyurethane prepolymer. The add-on was targeted for between 65 - 75%. A test cylinder was formed by wetting the coated reinforced scrim and wrapping five and eight layers of the tape around a 6,98 cm (2 3/4 inch) diameter dowel. The samples were aged for 24 hours prior to the crush evaluation. The crush strength was determined as in Example V.

**Table VIII**

| Layers | Strength of Cylinder kg (pounds) |
|---|---|
| 8 | 36,8 (81.17) |
| 5 | 16,9 (37.47) |

### Example IX

A nonwoven was formed on a 0,036 kg/m² (1.1 ounce per square yard) elastomeric scrim. The polyester staple fiber was entangled within the scrim while supported by a designed backing to topographically direct the filaments to a desired shape as disclosed in U. S. Patent 5,098,764. The scrim reinforced nonwoven was coated with a water hardenable polyurethane prepolymer. The add-on was targeted for between 70% and 75%. A test cylinder was formed by wetting the coated reinforced scrim and wrapping three to eight layers of the tape around a 6,98 cm (2 3/4 inch) diameter dowel. The samples were aged for 24 hours prior to the crush evaluation. The crush strength was determined as in Example V.

**Table IX**

| Layers | Strength of Cylinder kg (pounds) | Weight of Cylinder (grams) |
|---|---|---|
| 8 | 31,2 (68.90) | 46.93 |
| 7 | 23,6 (52.06) | 39.58 |
| 5 | 16,9 (37.39) | 34.65 |
| 4 | 10,4 (23.03) | 28.94 |
| 3 | 3,1 (6.92) | 17.64 |

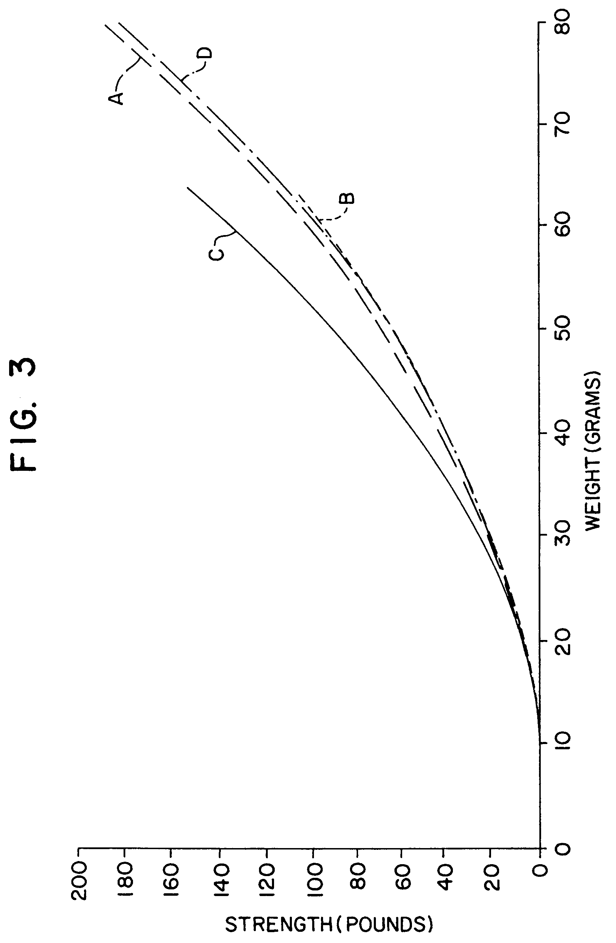

The strength of the casting tapes of the present invention is comparable to other polyurethane casting tapes. Fig. 3 is a graph of the crush strength in pounds to the weight, in grams, of test cylinders made with different substrates.

In Fig.3, Curve A is a casting tape made with a knitted substrate containing polyester and rubber yarns. Curve B is a casting tape made with a knitted polyester substrate with an extensibility of about 10 percent. Curve C is a casting tape made with a knitted polyester substrate with an extensibility of between 10 and 20 percent. Curve D is a casting tape made with the substrate of Example III of the present application. These curves show that casts made with the low cost casting tapes of the present invention is comparable in strength to casts made with conventional casting tape with knitted polyester substrates.

## Claims

1. An orthopedic casting tape comprising a substrate impregnated with a hardenable material, said substrate comprising a non-woven composite including a fibrous web (10) and a net reinforcing layer (15) secured to said fibrous web, said net reinforcing layer comprising a plurality of intersecting strands (21) having openings (20) between adjacent strands, said fibrous web comprising fibers (23) having a length of from 0,635 to 10,16 cm (.25 to 4 inches) and from 1 to 100 denier per fiber, and a weight of from 0,0083 to 0,33 kg/m² (0.25 to 10 ounces per square yard), said substrates have an extensibility of at least 5 percent in the length direction of the substrate.

2. The casting tape of claim 1 in which the net reinforcing layer comprises elastomeric strands.

3. The casting tape of claim 2 in which the substrate has a recovery after extension of at least 65 percent.

4. The casting tape of claim 2 in which the elastomeric strands comprise a blend of a polyolefin and a plastomer.

5. An orthopedic support material comprising a substrate impregnated with a water reactive polyurethane prepolymer material, said substrate comprising a non-woven composite including an elastomeric net reinforcing layer having opposed major surfaces and a fibrous web affixed to at least one major surface of said net, said fibrous web comprising a carded web of polyester fibers, said carded web being secured to said net reinforcing layer by needle punching the fibers of the carded web through said net, said substrate having a lengthwise extensibility of at least 5 percent, and a recovery of at least 65 percent, said elastomeric net comprising a thermoplastic elastomer.

6. The orthopedic support material of claim 5 in which the substrate has a recovery of at least 80 percent.

7. The orthopedic support material of claim 5 in which the water reactive polyurethane prepolymer is coated on the substrate at an add on level of between 45 and 80 weight percent based on the weight of the casting tape.

8. The orthopedic support material of claim 5 in which the elastomer net is composed of filaments of a blend of a polyolefin and a plastomer.

9. The orthopedic support material of claim 7 in which the add on level of the polyurethane prepolymer is between 65 percent and 75 percent.

10. The orthopedic support material of claim 5 in which the fibers in the substrate are dyed polyester fibers.

## Patentansprüche

1. Orthopädische Binde für einen steifen Verband mit einem Substrat, das mit einem härtbaren Material imprägniert ist, wobei das Substrat einen nicht gewebten Verbundstoff aufweist, der ein Faservlies (10) und eine netzförmige Verstärkungsschicht (15) aufweist, die mit dem Faservlies verbunden ist, wobei die netzförmige Verstärkungsschicht mehrere sich kreuzende Stränge (21) mit Öffnungen (20) zwischen benachbarten Strängen aufweist, das Faservlies Fasern (23) mit einer Länge von 0,635 cm bis 10,16 cm (0,25 Zoll bis 4 Zoll), 1 bis 100 Denier pro Faser und einem Gewicht von 0,0083 kg/m² bis 0,33 kg/m² (0,25 bis 10 ounces pro square yard) aufweist und das Substrat eine Dehnbarkeit von mindestens 5% in der Längsrichtung des Substrats besitzt.

2. Binde für einen steifen Verband nach Anspruch 1, bei der die netzförmige Verstärkungsschicht Elastomerstränge umfaßt.

3. Binde für einen steifen Verband nach Anspruch 2, bei der das Substrat ein Erholungsvermögen nach einer Dehnung von mindestens 65 % besitzt.

4. Binde für einen steifen Verband nach Anspruch 2, bei der die Elastomerstränge eine Mischung aus einem Polyolefin und einem Plastomer enthalten.

5. Orthopädisches Stützmaterial mit einem Substrat, das mit einem mit Wasser reaktiven Polyurethan - Vorpolymermaterial imprägniert ist, wobei das Substrat einen nicht gewebten Verbundstoff mit einer netzförmigen Elastomer - Verstärkungsschicht, die einander gegenüberliegende Hauptoberflächen besitzt, und einem Faservlies aufweist, das mit mindestens einer Hauptoberfläche des Netzes verbunden ist, wobei das Faservlies ein kardiertes Vlies aus Polyesterfasern umfaßt, das an der netzförmigen Verstärkungsschicht dadurch befestigt ist, daß die Fasern des kardierten Vlieses durch das Netz vernadelt sind, wobei das Substrat eine Längenausdehnbarkeit von mindestens 5% und ein Erholungsvermögen von mindestens 65 % aufweist und das Elastomernetz ein thermoplastisches Elastomer aufweist.

6. Orthopädisches Stützmaterial nach Anspruch 5, bei dem das Substrat ein Erholungsvermögen von mindestens 80 % besitzt.

7. Orthopädisches Stützmaterial nach Anspruch 5, bei dem das mit Wasser reaktive Polyurethan - Vorpolymer auf das Substrat mit einer Auftragsmenge zwischen 45 Gewichtsprozent und 80 Gewichtsprozent, bezogen auf das Gewicht der Binde, beschichtet ist.

8. Orthopädisches Stützmaterial nach Anspruch 5, bei dem das Elastomernetz aus Filamenten aus einer Mischung eines Polyolefins und eines Plastomers aufgebaut ist.

9. Orthopädisches Stützmaterial nach Anspruch 7, bei dem die Auftragsmenge des Polyurethan - Vorpolymers zwischen 65 % und 75 % liegt.

10. Orthopädisches Stützmaterial nach Anspruch 5, bei dem die Fasern in dem Substrat gefärbte Polyesterfasern sind.

## Revendications

1. Ruban pour plâtre orthopédique comprenant un substrat imprégné avec une matière durcissable, ledit substrat comprenant un matériau composite non tissé comprenant une bande fibreuse (10) et une couche de renfort formant filet (15) fixée à ladite bande fibreuse, ladite couche de renfort formant filet comprenant une pluralité de brins entrecroisés (21) ayant des ouvertures (20) entre les brins adjacents, ladite bande fibreuse comprenant des fibres (23) ayant une longueur comprise entre 0,635 et 10,16 cm (0,25 et 4 pouces) et ayant un titrage de fibre compris entre 1 et 100 deniers par fibre, et un poids compris entre 0,0083 et 0,33 kg/m2 (0,25 et 10 oz par yard carré), ledit substrat ayant une extensibilité d'au moins 5 % dans le sens de la longueur du substrat.

2. Ruban pour plâtre selon la revendication 1, dans lequel la couche de renfort formant filet comprend des brins élastomères.

3. Ruban pour plâtre selon la revendication 2, dans lequel le substrat revient en place après extension dans une proportion d'au moins 65 %.

4. Ruban pour plâtre selon la revendication 2, dans lequel les brins élastomères comprennent un mélange d'une polyoléfine et d'un plastomère.

5. Matériau de support orthopédique comprenant un substrat imprégné avec une matière prépolymère polyuréthane réagissant à l'eau, ledit substrat comprenant un matériau composite non tissé comprenant une couche de renfort formant filet élastomère ayant des surfaces principales opposées et une bande fibreuse fixée sur au moins une surface principale dudit filet, ladite bande fibreuse comprenant une bande cardée de fibres polyester, ladite bande cardée étant fixée à ladite couche de renfort formant filet en poinçonnant avec une aiguille les fibres de la bande cardée à travers ledit filet, ledit substrat ayant une extensibilité dans le sens de la longueur d'au moins 5 %, et revenant en place dans une proportion d'au moins 65 %, ledit filet élastomère comprenant un élastomère thermoplastique.

6. Matériau de support orthopédique selon la revendication 5, dans lequel le substrat revient en place dans une proportion d'au moins 80 %.

7. Matériau de support orthopédique selon la revendication 5, dans lequel le substrat est revêtu par le prépolymère polyuréthane réagissant à l'eau à un niveau supplémentaire compris entre 45 et 80 % en poids sur la base du poids du ruban pour plâtre.

8. Matériau de support orthopédique selon la revendication 5, dans lequel le filet élastomère se compose de filaments constitués d'un mélange d'une polyoléfine et d'un plastomère.

9. Matériau de support orthopédique selon la revendication 7, dans lequel le niveau supplémentaire du prépolymère polyuréthane est compris entre 65 et 75 %.

10. Matériau de support orthopédique selon la revendication 5, dans lequel les fibres dans le substrat sont des fibres polyester teintes.
